# EUROPEAN PATENT APPLICATION

(11) **EP 4 697 338 A1**
(43) Date of publication of application: **18.02.2026**
(21) Application number: 24195051.8
(22) Date of filing: 16.08.2024
(51) Int. Cl.: G16H 10/60, G16H 40/67, H04W 12/06

(54) **HEALTH MONITORING SYSTEM, METHOD AND APPARATUS**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application discloses a health monitoring system, a method and an apparatus. The health monitoring system includes a first terminal device 11 of a first user, a second terminal device 12 of a second user, a health monitoring device, and a server. The first terminal device 11 obtains a unique activation code from the server, and the server associates the unique activation code with first user information. The second terminal device 12, as an auxiliary device, obtains the unique activation code of the first terminal device 11, awakens the health monitoring device, and sends the obtained identification information of the health monitoring device and the unique activation code to the server. The server downlinks the identification information of the health monitoring device to the first terminal device 11. The first terminal device 11 establishes a connection to the health monitoring device, receives and displays health monitoring data sent by the health monitoring device. With the second terminal device 12 as an auxiliary device, the activation, connection and continuous monitoring of the health monitoring device are completed on the premise that the first terminal device 11 is operated as little as possible.

## Description

### TECHNICAL FIELD

The present application relates to the technical field of health monitoring, in particular to a health monitoring system, a method and an apparatus.

### BACKGROUND

The health monitoring device is increasingly widely used in public places such as hospitals and nursing homes. The health monitoring device worn by the user may collect the user's health monitoring data in real time or regularly. The user may view the health monitoring data collected by the health monitoring device through the terminal device. At least one health monitoring device communicate with the health monitoring software in the terminal device, and then upload the health monitoring data to the server through the health monitoring software, to realize health monitoring. The health monitoring device may be but not limited to a blood glucose monitoring device, an analyte monitoring device, an analyte sensor, a glucose detection sensor, and a general inspection and monitoring device. The inspection and monitoring device includes height and weight meter, sphygmomanometer, blood glucose meter, oximeter, pedometer, thermometer, body fat analyzer, BMI monitor, skinfold caliper, fetal heart monitor, heart rate monitor, muscle tester, and electronic spine measuring instrument.

The elderly accounts for a large proportion in the age distribution of users of health monitoring device. Due to many problems with the use of software by the elderly, they generally do not know how to use or do not want to use software products. How to complete the activation, connection and continuous monitoring of health monitoring devices with as little software operation by the elderly as possible is an urgent technical problem at present.

### SUMMARY

The present application provides a health monitoring system, a method and an apparatus to provide a technical solution for assisting in activating the health monitoring device and implementing the health monitoring.

According to a first aspect, the present application provides a health monitoring system, where the system includes a first terminal device of a first user, a second terminal device of a second user, a health monitoring device, and a server;
the first terminal device is configured to obtain a unique activation code from the server and generate a QR code containing the unique activation code, and the server is configured to associate the unique activation code with first user information;
the second terminal device is configured to obtain the unique activation code by scanning the QR code, awaken the health monitoring device, obtain identification information of the health monitoring device, and send the identification information of the health monitoring device and the unique activation code to the server;
the server is configured to downlink the identification information to the first terminal device;
the first terminal device is configured to establish a connection to the health monitoring device based on the identification information, receive and display health monitoring data sent by the health monitoring device.

According to a second aspect, the present application provides a health monitoring method, where the method is applied to a second terminal device and includes:
obtaining the unique activation code by scanning the QR code, awakening the health monitoring device, obtaining identification information of the health monitoring device, and sending the identification information of the health monitoring device and the unique activation code to the server; the server downlinks the identification information to the first terminal device; the first terminal device establishes a connection to the health monitoring device based on the identification information, receives and displays health monitoring data sent by the health monitoring device; the QR code is a QR code generated by the first terminal device and containing the unique activation code.

According to a third aspect, the present application provides a health monitoring method, where the method is applied to a first terminal device and includes:
obtaining a unique activation code from a server and generating a QR code containing the unique activation code; establishing a connection to a health monitoring device based on identification information of the health monitoring device, receiving and displaying health monitoring data sent by the health monitoring device; a second terminal device obtains the unique activation code by scanning the QR code, awakens the health monitoring device, obtains the identification information of the health monitoring device, and sends the identification information to the server; the server downlinks the identification information to the first terminal device.

According to a fourth aspect, the present application provides a health monitoring apparatus, where the apparatus is applied to a second terminal device and includes:
a first health monitoring unit, configured to obtain the unique activation code by scanning the QR code, awaken the health monitoring device, obtain identification information of the health monitoring device, and send the identification information of the health monitoring device and the unique activation code to the server; the server downlinks the identification information to the first terminal device; the first terminal device establishes a connection to the health monitoring device based on the identification information, receives and displays health monitoring data sent by the health monitoring device; the QR code is a QR code generated by the first terminal device and containing the unique activation code.

According to a fifth aspect, the present application provides a health monitoring apparatus, where the apparatus is applied to a first terminal device and includes:
a second health monitoring unit, configured to obtain a unique activation code from a server and generate a QR code containing the unique activation code; establish a connection to a health monitoring device based on identification information of the health monitoring device, receive health monitoring data sent by the health monitoring device, and display the health monitoring data; a second terminal device obtains the unique activation code by scanning the QR code, awakens the health monitoring device, obtains the identification information of the health monitoring device, and sends the identification information to the server; the server downlinks the identification information to the first terminal device.

According to a sixth aspect, the present application provides a terminal device, where it includes a processor, a communication interface, a memory, and a communication bus, and the processor, the communication interface and the memory communicate with each other through the communication bus;
the memory is configured to store a computer program;
the processor is configured to implement the steps of the method when executing the program stored in the memory.

According to a seventh aspect, the present application provides a computer-readable storage medium, where a computer program is stored on the computer-readable storage medium, and the computer program is executed by a processor to implement the steps of the method.

The above technical solutions have the following advantages or beneficial effects:
In the present application, the health monitoring system includes a first terminal device of a first user, a second terminal device of a second user, a health monitoring device, and a server; the first terminal device is configured to obtain a unique activation code from the server and generate a QR code containing the unique activation code, and the server is configured to associate the unique activation code with first user information; the second terminal device is configured to obtain the unique activation code by scanning the QR code, awaken the health monitoring device, obtain identification information of the health monitoring device, and send the identification information of the health monitoring device and the unique activation code to the server; the server is configured to downlink the identification information to the first terminal device; the first terminal device is configured to establish a connection to the health monitoring device based on the identification information, receive and display health monitoring data sent by the health monitoring device. In the present application, the awakening of the health monitoring device and the unique binding of server-side information and device connection between the health monitoring device and the first terminal device of the first user are implemented through the second terminal device of the second user, and the first terminal device may receive and display the health monitoring data sent by the health monitoring device, while the second terminal device implements the auxiliary monitoring of the first user. For example, the first user is an elderly user and the second user is another user such as a relative user and others. In the present application, the first terminal device of the elderly user connects to the health monitoring device by operating the second terminal device by the relative user to realize the health monitoring of the elderly user. The elderly user may view the health monitoring data through the first terminal device. The activation, connection and continuous monitoring of the health monitoring device are completed on the premise that the first terminal device is operated as little as possible.

The whole process improves convenience, security, and timeliness.

Convenience: The first user only needs to present the activation QR code to complete the subsequent activation of the health monitoring device and the second user monitoring.

Security: The QR code presented by the first user is unique and time-sensitive to ensure the security of the auxiliary activation process.

Timeliness: The mqtt protocol is used for data interaction between the first terminal device and the second terminal device, so that when there is new data, it can be pushed to the corresponding device in a timely manner, and the consumption of server resources and network bandwidth by regular polling is avoided.

### BRIEF DESCRIPTION OF DRAWINGS

To illustrate the technical solutions in embodiments of the present application more clearly, the drawings to be used for describing the embodiments are introduced briefly in the following. Apparently, the drawings in the following description are only some embodiments of the present application, and persons of ordinary skill in the art can derive other drawings from these drawings without creative efforts.
FIG. 1 is a structural diagram of a health monitoring system according to the present application;
FIG. 2 is a schematic diagram of a process of obtaining a unique activation code according to the present application;
FIG. 3 is a schematic diagram of a process of mutual authentication between a health monitoring device and a first terminal device according to the present application;
FIG. 4 is a schematic diagram of a health monitoring process according to the present application;
FIG. 5 is a schematic diagram of a health monitoring process according to the present application;
FIG. 6 is a flow chart of activation and connection of a health monitoring device according to the present application;
FIG. 7 is a flow chart of sharing health monitoring data by a first terminal device according to the present application;
FIG. 8 is a structural diagram of a terminal device according to the present application.

### DESCRIPTION OF EMBODIMENTS

To make the objectives and embodiments of the present application clearer, the exemplary embodiments of the present application will be clearly and completely described below in combination with the drawings in the exemplary embodiments of the present application. Obviously, the exemplary embodiments described are only some rather than all of the embodiments of the present application.

It should be noted that the brief description of terms in the present application is only for the convenience of understanding the embodiments described next, and does not intend to limit the embodiments of the present application. Unless otherwise stated, these terms shall be construed according to their ordinary and usual meanings.

Unless otherwise indicated, the terms "first", "second" and "third" in the specification, claims and drawings of the present application are used to distinguish similar or homogeneous objects or entities, and do not necessarily mean to limit a specific sequence or precedence. It should be understood that the terms so used are interchangeable where appropriate.

The terms "include" and "have" and any variations thereof are intended to cover but not exclusively include, for example, a product or device that includes a range of components need not be limited to all components expressly listed, but may include other components not expressly listed or inherent to the product or device.

The term "module" refers to any known or later developed hardware, software, firmware, artificial intelligence, fuzzy logic, or combination of hardware or/and software code capable of performing the functions associated with the component.

Finally, it should be noted that the above embodiments are only used to illustrate the technical solutions of the present application and not to limit them. Although the present application is described in detail with reference to the above embodiments, those skilled in the art shall understand that they can still modify the technical solutions recorded in the above embodiments or equivalently replace part or all of the technical features therein. These modifications or replacements do not depart from the scope of the technical solutions in embodiments of the present application in essence.

For ease of explanation, the above description is made in connection with specific embodiments. However, the above exemplary discussion is not intended to be exhaustive or to limit embodiments to the specific forms disclosed above. Various modifications and variations may be obtained according to the above teachings. The above embodiments are selected and described to better explain the principles and practical applications, so that those skilled in the art can make better use of the embodiments and various variants suitable for specific use considerations.

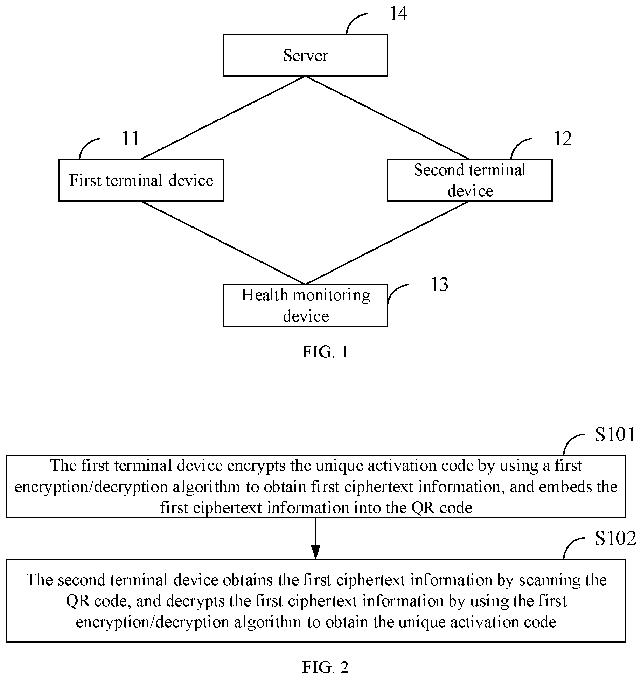

FIG. 1 is a structural diagram of a health monitoring system according to the present application, where the system includes a first terminal device 11 of a first user, a second terminal device 12 of a second user, a health monitoring device 13, and a server 14;
the first terminal device 11 is configured to obtain a unique activation code from the server 14 and generate a QR code containing the unique activation code, and the server 14 is configured to associate the unique activation code with first user information;
the second terminal device 12 is configured to obtain the unique activation code by scanning the QR code, awaken the health monitoring device 13, obtain identification information of the health monitoring device 13, and send the identification information of the health monitoring device 13 and the unique activation code to the server 14;
the server 14 is configured to downlink the identification information to the first terminal device 11;
the first terminal device 11 is configured to establish a connection to the health monitoring device 13 based on the identification information, receive health monitoring data sent by the health monitoring device 13, and display the health monitoring data.

In the present application, the first user is different from the second user. The first user may be the monitored object of health monitoring, and the second user may be a relative of the first user or a medical staff. The first terminal device 11 is a device used by the first user, and may be a mobile phone, tablet computer, and personal digital assistant (PDA, a portable electronic device). The second terminal device 12 is a device used by the second user, and may be a mobile terminal device such as mobile phone, tablet computer, PDA, and notebook computer. The server 14 may be an independent physical server or a cloud server for basic cloud computing services such as cloud database and cloud storage.

The health monitoring device can connect to the first terminal device and the second terminal device through Bluetooth, and the server can wirelessly connect to the first terminal device 11 and the second terminal device 12, for example, through Bluetooth or WIFI connection.

The second terminal device 12 is configured to awaken the health monitoring device. Optionally, the second terminal device 12 may awaken the health monitoring device through the near field communication (NFC) technology. The second terminal device 12 is further configured to obtain identification information of the health monitoring device. The identification information of the health monitoring device may be the media access control address (MAC address, a physical address) information and device serial number information of the health monitoring device. In addition, the second terminal device 12 may connect to the health monitoring device through NFC, or the identification of the NFC link, that is, the NFC ID of the health monitoring device, may be used as the identification information of the health monitoring device.

The first terminal device 11 obtains a unique activation code from the server to generate a QR code containing the unique activation code, and the server associates the unique activation code with first user information. The second terminal device 12 obtains the unique activation code by scanning the QR code, awakens the health monitoring device, obtains identification information of the health monitoring device, and sends the identification information of the health monitoring device and the unique activation code to the server. The server downlinks the identification information to the first terminal device 11 to establish a binding relationship between the first terminal device and the health monitoring device. The first terminal device 11 may search for the health monitoring device based on the identification information after receiving the identification information to establish a connection to the health monitoring device. Optionally, the first terminal device 11 establishes a wireless connection, such as Bluetooth connection, to the health monitoring device. The health monitoring device is configured to collect health monitoring data of a user wearing the health monitoring device, and the user wearing the health monitoring device may be the first user or any other user. In the present application, if the user wearing the health monitoring device is the first user, the health monitoring device collects health monitoring data of the first user and sends the health monitoring data of the first user to the first terminal device 11 of the first user, so that the first user may view his/her own physical condition through the first terminal device. If the user wearing the health monitoring device is another user, the health monitoring device collects health monitoring data of another user and sends the health monitoring data of another user to the first terminal device 11 of the first user, so that the first user may view the physical condition of another user through the first terminal device. The first terminal device 11 may include a display screen, and the first terminal device 11 may display the health monitoring data on the display screen after receiving the health monitoring data.

In the present application, the awakening of the health monitoring device and the unique binding of server-side information and device connection between the health monitoring device and the first terminal device 11 of the first user are implemented through the second terminal device 12 of the second user, and the first terminal device 11 may receive and display the health monitoring data sent by the health monitoring device, while the second terminal device 12 implements the auxiliary monitoring of the first user. For example, the first user is an elderly user and the second user is another user such as a relative user and others. In the present application, the first terminal device 11 of the elderly user connects to the health monitoring device by operating the second terminal device 12 by the relative user to realize the health monitoring of the elderly user. The elderly user may view the health monitoring data through the first terminal device 11. The activation, connection and continuous monitoring of the health monitoring device are completed on the premise that the first terminal device 11 is operated as little as possible.

The whole process has convenience, security, and timeliness. Convenience: The first user only needs to present the activation QR code to complete the subsequent activation of the health monitoring device and the second user monitoring.

Security: The QR code presented by the first user is unique and time-sensitive to ensure the security of the auxiliary activation process.

Timeliness: The mqtt (Messaage Queuing Telemetry Transport) protocol is used for data interaction between the first terminal device 11 and the second terminal device 12, so that when there is new data between them, one end device can be pushed to the other device in a timely manner, and the consumption of server resources and network bandwidth by regular polling is avoided.

In the present application, the server verifies the identification information and the unique activation code before downlinking the identification information to the first terminal device 11, and associates the identification information of the health monitoring device with the first user information based on the unique activation code after the verification is passed, to realize the binding between the health monitoring device and the first user and downlink the identification information to the first terminal device 11.

The first terminal device 11 may log in to the system with the account number and password or the mobile phone number and verification code; the server verifies user identity information and returns an access token after the verification is passed; the first terminal device 11 carries the access token on the subsequent access to the server; the first terminal device requests a unique activation code from the server when the user selects auxiliary activation. The first terminal device 11 embeds the unique activation code into the QR code, and the second terminal device 12 scans the QR code through an application (APP) to obtain the unique activation code. The unique activation code may be a universally unique identifier (UUID) or a unique code generated by using a snowflake algorithm.

The server verifies the identification information and the unique activation code before downlinking the identification information to the first terminal device 11, where the verification is passed if the following conditions are met simultaneously: (1) the health monitoring device corresponding to the current identification information is not activated and the health monitoring device does not expire; (2) the current unique activation code is within the validity period and does not become invalid, and the current unique activation code within the validity period means that the time difference between the current time and the binding time of the unique activation code and the user information is less than the set time threshold; (3) the unique activation code is decrypted normally. The server downlinks the identification information to the first terminal device 11 after the server verifies the identification information and the unique activation code and the verification is passed.

To obtain more accurate information, the first terminal device 11 is further configured to encrypt the unique activation code by using a first encryption/decryption algorithm to obtain first ciphertext information, and embed the first ciphertext information into the QR code; the second terminal device 12 is specifically configured to obtain the first ciphertext information by scanning the QR code, and decrypt the first ciphertext information by using the first encryption/decryption algorithm to obtain the unique activation code. The first encryption/decryption algorithm is a symmetrical encryption/decryption algorithm or an asymmetrical encryption/decryption algorithm. The first terminal device 11 encrypts the unique activation code with an encryption key in the first encryption/decryption algorithm to obtain the first ciphertext information, and embeds the first ciphertext information into the QR code, so that the second terminal device 12 obtains the first ciphertext information by scanning the QR code, and then decrypts the first ciphertext information with a decryption key in the first encryption/decryption algorithm to obtain the unique activation code.

FIG. 2 is a schematic diagram of a process of obtaining a unique activation code according to the present application, where the process includes the following steps:
S 1 01: The first terminal device 11 encrypts the unique activation code by using a first encryption/decryption algorithm to obtain first ciphertext information, and embeds the first ciphertext information into the QR code.
S102: The second terminal device 12 obtains the first ciphertext information by scanning the QR code, and decrypts the first ciphertext information by using the first encryption/decryption algorithm to obtain the unique activation code.

In the present application, the device authentication is performed after the health monitoring device connects to the first terminal device 11 and before the health monitoring device sends the health monitoring data to the first terminal device. In the present application, a mutual authentication method is used. The health monitoring device and the first terminal device 11 verify the validity of each other. After successful mutual authentication, the health monitoring device sends the health monitoring data to the first terminal device 11.

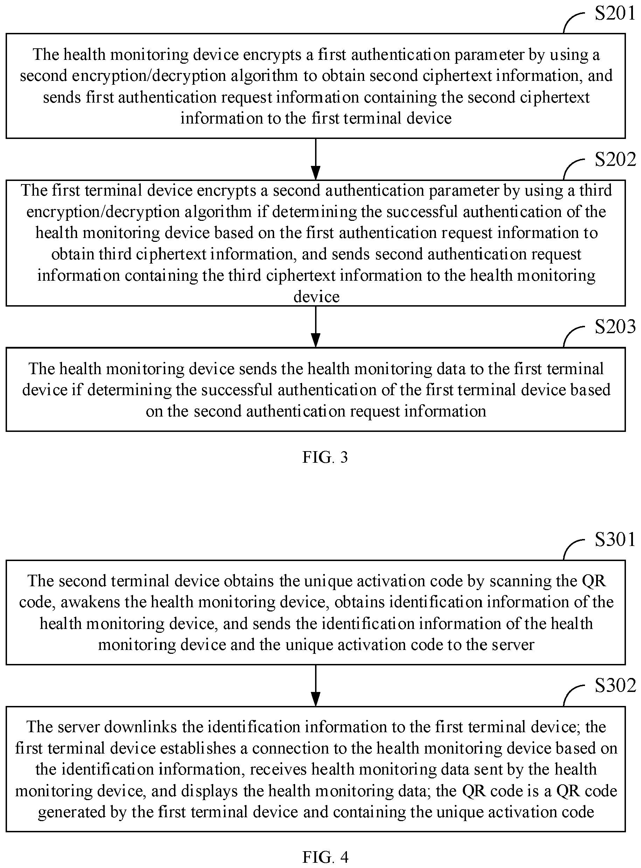

FIG. 3 is a schematic diagram of a process of mutual authentication between a health monitoring device and a first terminal device 11 according to the present application, where the process includes the following steps:
S201: The health monitoring device encrypts a first authentication parameter by using a second encryption/decryption algorithm to obtain second ciphertext information, and sends first authentication request information containing the second ciphertext information to the first terminal device 11.
S202: The first terminal device 11 encrypts a second authentication parameter by using a third encryption/decryption algorithm if determining the successful authentication of the health monitoring device based on the first authentication request information to obtain third ciphertext information, and sends second authentication request information containing the third ciphertext information to the health monitoring device.
S203: The health monitoring device sends the health monitoring data to the first terminal device 11 if determining the successful authentication of the first terminal device 11 based on the second authentication request information.

The health monitoring device and the first terminal device 11 both save the same plurality of sets of authentication parameters. For example, the health monitoring device and the first terminal device 11 both save the same 6 sets of authentication parameters, and each set of authentication parameters includes key information and key identifier information. The health monitoring device randomly selects one set of authentication parameters from the plurality of sets of authentication parameters as a first authentication parameter. The health monitoring device encrypts the first authentication parameter by using a second encryption/decryption algorithm to obtain second ciphertext information. The second encryption/decryption algorithm includes but is not limited to SHA 256 hash algorithm, ECDH key agreement algorithm, and AES symmetric encryption algorithm. The health monitoring device generates first authentication request information based on the second ciphertext information, and the first authentication request information contains the second ciphertext information. The health monitoring device sends the first authentication request information to the first terminal device 11.

The first terminal device 11 obtains the contained second ciphertext information by parsing the first authentication request information after receiving the first authentication request information. The first terminal device decrypts the second ciphertext information by using the second encryption/decryption algorithm to obtain the first authentication parameter, and obtains first key information and corresponding first key identifier information in the first authentication parameter. The first terminal device obtains locally stored second key information based on the first key identifier information. That is, the locally stored second key information corresponding to the first key identifier information is obtained. Then, the first terminal device determines whether the first key information and the second key information are the same. If they are the same, the authentication of the health monitoring device is successful. If they are not the same, the authentication of the health monitoring device fails.

The first terminal device 11 encrypts a second authentication parameter by using a third encryption/decryption algorithm if determining the successful authentication of the health monitoring device based on the first authentication request information to obtain third ciphertext information, and sends second authentication request information containing the third ciphertext information to the health monitoring device. The first terminal device 11 randomly selects one set of authentication parameters from the plurality of sets of authentication parameters as a second authentication parameter. The first terminal device 11 encrypts the first authentication parameter by using the third encryption/decryption algorithm to obtain the second ciphertext information. It should be noted that the second authentication parameter and the first authentication parameter can be the same or different. The third encryption/decryption algorithm includes but is not limited to SHA 256 hash algorithm, ECDH key agreement algorithm, and AES symmetric encryption algorithm. The third encryption/decryption algorithm and the second encryption/decryption algorithm may be the same or different. The first terminal device 11 generates the second authentication request information based on the third ciphertext information, and the second authentication request information contains the third ciphertext information. The first terminal device 11 sends the second authentication request information to the health monitoring device.

The health monitoring device obtains the contained third ciphertext information by parsing the second authentication request information after receiving the second authentication request information. The health monitoring device decrypts the third ciphertext information by using the third encryption/decryption algorithm to obtain the second authentication parameter, and obtains second key information and corresponding second key identifier information in the second authentication parameter. The health monitoring device obtains locally stored fourth key information based on the second key identifier information. That is, the locally stored fourth key information corresponding to the second key identifier information is obtained. Then, the health monitoring device determines whether the third key information and the fourth key information are the same. If they are the same, the authentication of the first terminal device 11 is successful. If they are not the same, the authentication of the first terminal device 11 fails. The health monitoring device sends the health monitoring data to the first terminal device 11 if determining the successful authentication of the first terminal device 11 based on the second authentication request information. If either the authentication of the first terminal device 11 or the authentication of the health monitoring device fails, the health monitoring device does not send the health monitoring data to the first terminal device 11.

In the present application, in order that the second user may view the health monitoring data of the monitored object through the second terminal device 12, the first terminal device is further configured to send the health monitoring data to the server; the server is further configured to send the health monitoring data to the second terminal device 12 for display. The second terminal device 12 might include a display screen, and the second terminal device 12 may display the health monitoring data on the display screen after receiving the health monitoring data.

The first terminal device 11 is further configured to send monitoring invitation information containing account information of the second terminal device 12 to the server; the server is further configured to send a download link of a data monitoring system to the second terminal device 12 if determining that the second terminal device 12 has not downloaded and logged in to the data monitoring system based on the account information, the second terminal device 12 may download and log in to the data monitoring system; send the monitoring invitation information to the second terminal device 12 if determining that the second terminal device has downloaded and logged in to the data monitoring system based on the account information; the server is further configured to send the health monitoring data to the second terminal device 12 for display after receiving an invitation acceptance instruction sent by the second terminal device.

The first terminal device 11 may also send the monitoring invitation information containing the account information of the second terminal device 12 to the server. The server saves the state information of each terminal device downloading and logging in to the data monitoring system, and the state information includes downloading and logging in to the data monitoring system and not downloading and logging in to the data monitoring system. The server sends a download link of the data monitoring system to the second terminal device 12 before sending the monitoring invitation information to the second terminal device 12 if determining that the second terminal device has not downloaded and logged in to the data monitoring system based on the account information. The server may send the download link of the data monitoring system to the second terminal device 12 by mail, short message or other means. The data monitoring system may be an application (APP) that monitors health data. The second user downloads and logs in to the data monitoring system by clicking the download link of the data monitoring system on the second terminal device 12. After the second terminal device downloads and logs in to the data monitoring system, the server sends the monitoring invitation information to the second terminal device. The second terminal device 12 may display an option of whether to accept the monitoring invitation on the display screen after receiving the monitoring invitation information. If the second user selects the option of accepting the monitoring invitation, the second terminal device 12 sends an invitation acceptance instruction to the server, and at this time, the server sends the health monitoring data to the second terminal device 12 for display. If the second user selects the option of not accepting the monitoring invitation, the second terminal device 12 sends an invitation rejection instruction to the server, and at this time, the server does not send the health monitoring data to the second terminal device for display.

FIG. 4 is a schematic diagram of a health monitoring process according to the present application, where the process includes the following steps:
S301: The second terminal device 12 obtains the unique activation code by scanning the QR code, awakens the health monitoring device, obtains identification information of the health monitoring device, and sends the identification information of the health monitoring device and the unique activation code to the server.
S302: The server downlinks the identification information to the first terminal device 11; the first terminal device 11 establishes a connection to the health monitoring device based on the identification information, receives and displays health monitoring data sent by the health monitoring device; the QR code is a QR code generated by the first terminal device and containing the unique activation code.

Obtaining the unique activation code by scanning the QR code includes:
The second terminal device obtains the first ciphertext information by scanning the QR code, and decrypting the first ciphertext information by using the first encryption/decryption algorithm to obtain the unique activation code; the first terminal device 11 encrypts the unique activation code by using the first encryption/decryption algorithm to obtain the first ciphertext information, and embeds the first ciphertext information into the QR code.

The unique activation code is a universally unique identifier or a unique code generated by using a snowflake algorithm;
sending the identification information of the health monitoring device and the unique activation code to the server; the server verifies the identification information and the unique activation code before downlinking the identification information to the first terminal device, where the verification is passed if the following conditions are met simultaneously: the health monitoring device corresponding to the current identification information is not activated and the health monitoring device does not expire; the current unique activation code is within the validity period and does not become invalid, and the current unique activation code within the validity period means that the time difference between the current time and the binding time of the unique activation code and the user information is less than the set time threshold; the unique activation code information is successfully decrypted.

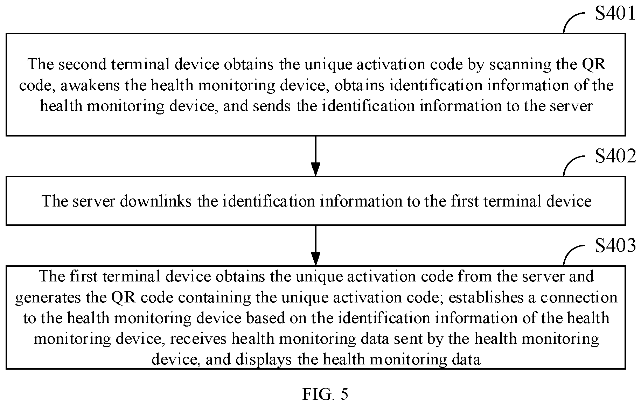

FIG. 5 is a schematic diagram of a health monitoring process according to the present application, where the process includes the following steps:
S401: The second terminal device 12 obtains the unique activation code by scanning the QR code, awakens the health monitoring device, obtains identification information of the health monitoring device, and sends the identification information to the server.
S402: The server downlinks the identification information to the first terminal device 11.
S403: The first terminal device 11 obtains the unique activation code from the server and generates the QR code containing the unique activation code; establishes a connection to the health monitoring device based on the identification information of the health monitoring device, receives and displays health monitoring data sent by the health monitoring device.

As shown in FIG.5, the method further includes:
The first terminal device 11 generates the QR code containing the unique activation code; the second terminal device 12 obtains the unique activation code by scanning the QR code, and sends the identification information and the unique activation code to the server; the server verifies the identification information and the unique activation code, and associates the identification information of the health monitoring device with first user information based on the unique activation code after the verification is passed, to realize the binding between the health monitoring device and a first user and downlink the identification information to the first terminal device 11.

The generating the QR code containing the unique activation code includes:
encrypting the unique activation code by using a first encryption/decryption algorithm to obtain first ciphertext information, and embedding the first ciphertext information into the QR code; the second terminal device 12 obtains the first ciphertext information by scanning the QR code, and decrypts the first ciphertext information by using the first encryption/decryption algorithm to obtain the unique activation code.

The method further includes:
The first terminal device 11 first authentication request information sent by the health monitoring device, where the health monitoring device encrypts a first authentication parameter by using a second encryption/decryption algorithm to obtain second ciphertext information, and sends the first authentication request information containing the second ciphertext information to the first terminal device 11;
encrypting a second authentication parameter by using a third encryption/decryption algorithm if the first terminal device determining the successful authentication of the health monitoring device based on the first authentication request information to obtain third ciphertext information, and sending second authentication request information containing the third ciphertext information to the health monitoring device; the health monitoring device sends the health monitoring data to the first terminal device if determining the successful authentication of the first terminal device 11 based on the second authentication request information.

The determining the successful authentication of the health monitoring device based on the first authentication request information includes:
The first terminal device 11 obtains the second ciphertext information contained in the first authentication request information, and decrypts the second ciphertext information by using the second encryption/decryption algorithm to obtain the first authentication parameter; obtaining first key information and corresponding first key identifier information in the first authentication parameter; obtaining locally stored second key information corresponding to the first key identifier information; determining the successful authentication of the health monitoring device if the first key information and the second key information are the same.

The method further includes:
The first terminal device 11 sends the health monitoring data to the server; the server sends the health monitoring data to the second terminal device for display.

The method includes:
The first terminal device 11 sends monitoring invitation information containing account information of the second terminal device to the server; the server sends a download link of a data monitoring system to the second terminal device 12 if determining that the second terminal device 12 has not downloaded and logged in to the data monitoring system based on the account information, the second terminal device 12 may download and log in to the data monitoring system; the server sends the monitoring invitation information to the second terminal device 12 if determining that the second terminal device has downloaded and logged in to the data monitoring system based on the account information; the server sends the health monitoring data to the second terminal device 12 for display after receiving an invitation acceptance instruction sent by the second terminal device.

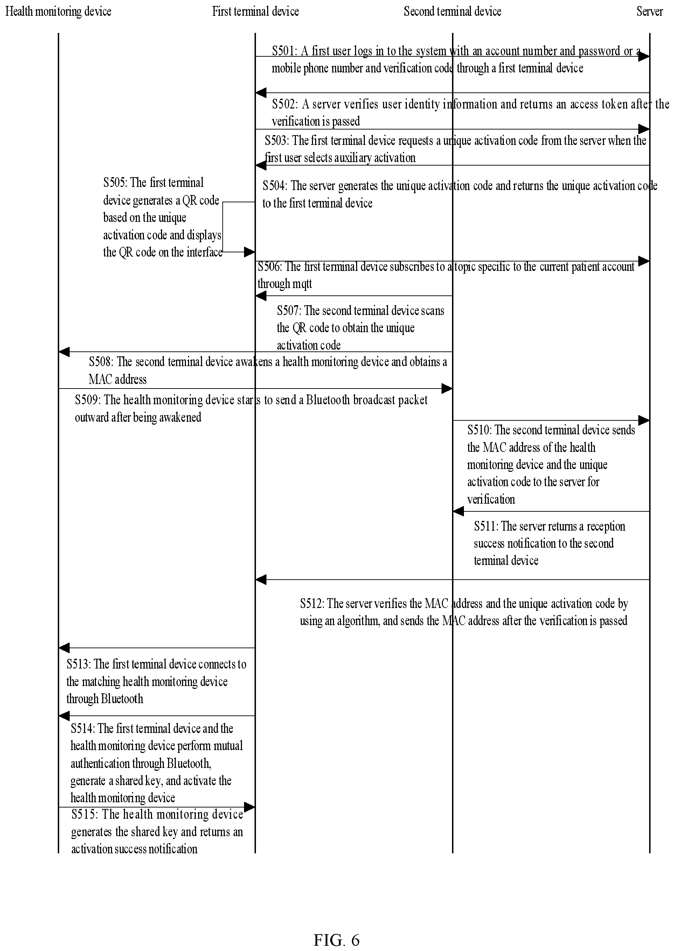

FIG. 6 is a flow chart of activation and connection of a health monitoring device according to the present application.
S501: a first user logs in to the system with an account number and password or a mobile phone number and verification code through a first terminal device 11;
S502: a server verifies user identity information and returns an access token to the first user after the verification is passed; the first terminal device carries the access token on the subsequent access to the server;
S503: the first terminal device requests a unique activation code from the server when the first user selects auxiliary activation;
S504: the server generates the unique activation code (the activation code may be a UUID or a unique code generated by using a snowflake algorithm), binds the unique activation code to the user account, records the binding time, and then returns the unique activation code to the first terminal device 11;
S505: the first terminal device 11 generates a QR code based on the unique activation code and displays the QR code on the interface;
S506: the first terminal device 11 subscribes to a topic specific to the current patient account through mqtt;
S507: a second user scans and identifies the QR code displayed on the first terminal device 11 through the camera of a second terminal device 12 to obtain the unique activation code, and saves it for subsequent use;
S508: the second user 12 awakens a health monitoring device through NFC of the second terminal device and obtains a MAC address of the health monitoring device;
S509: the health monitoring device starts to send a Bluetooth broadcast packet outward after being awakened;
S510: the second terminal device 12 sends the MAC address of the health monitoring device and the unique activation code to the server for verification;
S511: the server returns a reception success notification to the second terminal device 12;
S512: the server verifies the MAC address and the unique activation code by using an algorithm to determine whether the health monitoring device can be activated, sets the unique activation code as invalid after the verification is passed, and sends the MAC address to the mqtt subject of the user account corresponding to the unique activation code, so that the first terminal device 11 may receive the MAC address and the server records the binding relationship between the account of the first terminal device and the account of the second terminal device 12. The following conditions need to be met simultaneously for the verification algorithm: (1) the health monitoring device corresponding to the current MAC address is not activated and the health monitoring device does not expire; (2) the current unique activation code is within the validity period (current time - binding time of the unique activation code < set time threshold) and does not become invalid; (3) the unique activation code is decrypted normally.
S513: the first terminal device 11 connects to the matching health monitoring device through Bluetooth after receiving the MAC address;
S514: the first terminal device 11 and the health monitoring device perform mutual authentication through Bluetooth, generate a shared key, and activate the health monitoring device;
S515: the health monitoring device generates the shared key and returns an activation success notification.

So far, the second terminal device 12 assists the first terminal device 11 to complete the activation and connection operation of the wearable health monitoring device through the sensor camera and NFC module, and ensures the security of the whole activation and connection process.

In the data transmission stage, the health monitoring device encrypts the health monitoring data with the shared key by using an AES algorithm, and then pushes the encrypted data to the first terminal device 11 through Bluetooth. The first terminal device 11 decrypts the encrypted data with the shared key after obtaining the data and sends the data to the server. The server inquires about the binding relationship between the account of the first terminal device 11 and the account of the second terminal device 12 after obtaining the health monitoring data uploaded by the App of the first terminal device, and downlinks the health monitoring data to the corresponding second terminal device through the mqtt protocol to provide auxiliary health monitoring capability for the second terminal device 12.

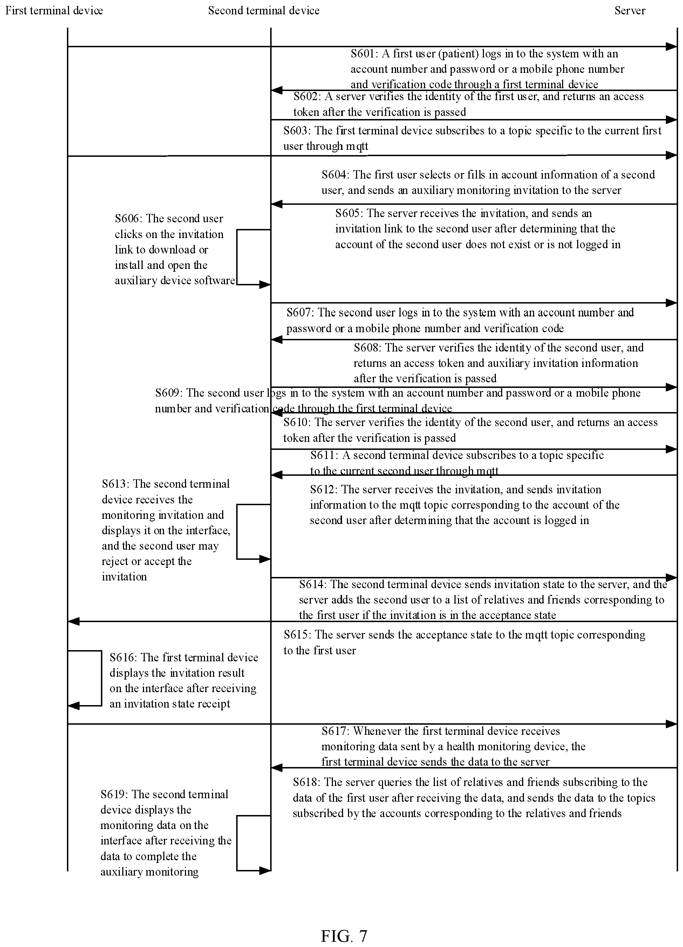

FIG. 7 is a flow chart of sharing health monitoring data by a first terminal device 11 according to the present application.
S601: a first user (patient) logs in to the system with an account number and password or a mobile phone number and verification code through a first terminal device 11;
S602: a server verifies the identity of the first user, and returns an access token after the verification is passed;
S603: the first terminal device 11 subscribes to a topic specific to the current first user through mqtt;
S604: the first user selects or fills in account information of a second user, and sends an auxiliary monitoring invitation to the server;
S605: the server receives the invitation, and sends an invitation link to the second user after determining that the account of the second user does not exist or is not logged in;
S606: the second user clicks on the invitation link to download or install and open the auxiliary device software;
after that, there are two situations: the first situation is that the second user does not download or log in to the auxiliary device software; at this time, the server receives the invitation, and sends the invitation link to the mailbox corresponding to the account of the second user by email or to the terminal device corresponding to the account of the second user by short message after determining that the account of the second user does not exist or is not logged in; the second user clicks on the invitation link after receiving the invitation, and downloads the auxiliary device software through the download link if the second user does not download and install the auxiliary device software and opens the auxiliary device software if the second user has installed the auxiliary device software;
S607: the second user logs in to the system with an account number and password or a mobile phone number and verification code;
S608: the server verifies the identity of the second user, and returns an access token and auxiliary invitation information after the verification is passed;
S609: the second user logs in to the system with an account number and password or a mobile phone number and verification code through the first terminal device 11;
the second situation is that the second user downloads and logs in to the auxiliary device software in advance; at this time, the second user logs in to the system with an account number and password or a mobile phone number and verification code;
S610: the server verifies the identity of the second user, and returns an access token after the verification is passed;
S611: a second terminal device subscribes to a topic specific to the current second user through mqtt;
S612: the server receives the invitation, and sends invitation information to the mqtt topic corresponding to the account of the second user after determining that the account is logged in, so that the second user receives the invitation information, and the invitation information includes account information of the first user;
S613: the second terminal device 12 receives the monitoring invitation and displays it on the interface, and the second user may reject or accept the invitation;
S614: after the second user makes the selection, the second terminal device sends first user information and invitation state to the server, and the server adds the second user to a list of relatives and friends corresponding to the first user for saving if the invitation is in the acceptance state;
S615: the server sends the acceptance state to the mqtt topic corresponding to the first user;
S616: the first terminal device 11 displays the invitation result on the interface after receiving an invitation state receipt;
S617: Whenever the first terminal device 11 receives monitoring data sent by a health monitoring device, the first terminal device sends the data to the server;
S618: the server saves the data after receiving the data, queries the list of relatives and friends subscribing to the data of the first user, and sends the data to the topics subscribed by the accounts corresponding to all relatives and friends of the first user through mqtt;
S619: the second terminal device 12 displays the monitoring data on the interface after receiving the data to complete the auxiliary monitoring.

In the health monitoring solution according to the present application, when the first terminal device 11 has no NFC or there is an obstacle in the use of the electronic device, the second user scans a QR code generated by the first terminal device 11 through the camera of the second terminal device 12, awakens the health monitoring device through NFC, and obtains the MAC address of the health monitoring device through NFC. The whole process has convenience, security, and timeliness.

Convenience: The first user only needs to present the invitation QR code to complete the subsequent activation of the health monitoring device and the second user monitoring.

Security: The QR code presented by the first user is unique and time-sensitive to ensure the security of the auxiliary activation process.

Timeliness: The mqtt protocol is used for data interaction between the first terminal device 11 and the second terminal device 12, so that when there is new subscription data, it can be pushed to the corresponding device in a timely manner, and the consumption of server resources and network bandwidth by regular polling is avoided.

The present application provides a health monitoring apparatus, where the apparatus is applied to the second terminal device 12 and includes:
a first health monitoring unit, configured to obtain the unique activation code by scanning the QR code, awaken the health monitoring device, obtain identification information of the health monitoring device, and send the identification information of the health monitoring device and the unique activation code to the server; the server downlinks the identification information to the first terminal device 11; the first terminal device establishes a connection to the health monitoring device based on the identification information, receives and displayshealth monitoring data sent by the health monitoring device; the QR code is a QR code generated by the first terminal device and containing the unique activation code;
a first health monitoring unit, configured to obtain the first ciphertext information by scanning the QR code, and decrypt the first ciphertext information by using the first encryption/decryption algorithm to obtain the unique activation code; the first terminal device 11 encrypts the unique activation code by using the first encryption/decryption algorithm to obtain the first ciphertext information, and embeds the first ciphertext information into the QR code;
the unique activation code is a universally unique identifier or a unique code generated by using a snowflake algorithm;
a first health monitoring unit, configured to send the identification information of the health monitoring device and the unique activation code to the server; the server verifies the identification information and the unique activation code before downlinking the identification information to the first terminal device 11, where the verification is passed if the following conditions are met simultaneously: the health monitoring device corresponding to the current identification information is not activated and the health monitoring device does not expire; the current unique activation code is within the validity period and does not become invalid, and the current unique activation code within the validity period means that the time difference between the current time and the binding time of the unique activation code and the user information is less than the set time threshold; the unique activation code information is successfully decrypted.

The present application provides a health monitoring apparatus, where the apparatus is applied to the first terminal device 11 and includes:
a second health monitoring unit, configured to obtain a unique activation code from a server and generate a QR code containing the unique activation code; establish a connection to a health monitoring device based on identification information of the health monitoring device, receive health monitoring data sent by the health monitoring device, and display the health monitoring data; the second terminal device 12 obtains the unique activation code by scanning the QR code, awakens the health monitoring device, obtains the identification information of the health monitoring device, and sends the identification information to the server; the server downlinks the identification information to the first terminal device;
a second health monitoring unit, configured to generate the QR code containing the unique activation code; the second terminal device 12 obtains the unique activation code by scanning the QR code, and sends the identification information and the unique activation code to the server; the server verifies the identification information and the unique activation code, and associates the identification information of the health monitoring device with first user information based on the unique activation code after the verification is passed, to realize the binding between the health monitoring device and a first user and downlink the identification information to the first terminal device 11;
a second health monitoring unit, configured to encrypt the unique activation code by using a first encryption/decryption algorithm to obtain first ciphertext information, and embed the first ciphertext information into the QR code; the second terminal device obtains the first ciphertext information by scanning the QR code, and decrypts the first ciphertext information by using the first encryption/decryption algorithm to obtain the unique activation code;
a second health monitoring unit, configured to receive first authentication request information sent by the health monitoring device, where the health monitoring device encrypts a first authentication parameter by using a second encryption/decryption algorithm to obtain second ciphertext information, and sends the first authentication request information containing the second ciphertext information to the first terminal device 11; encrypt a second authentication parameter by using a third encryption/decryption algorithm if determining the successful authentication of the health monitoring device based on the first authentication request information to obtain third ciphertext information, and send second authentication request information containing the third ciphertext information to the health monitoring device; the health monitoring device sends the health monitoring data to the first terminal device if determining the successful authentication of the first terminal device 11 based on the second authentication request information;
a second health monitoring unit, configured to obtain the second ciphertext information contained in the first authentication request information, and decrypt the second ciphertext information by using the second encryption/decryption algorithm to obtain the first authentication parameter; obtain first key information and corresponding first key identifier information in the first authentication parameter; obtain locally stored second key information corresponding to the first key identifier information; determine the successful authentication of the health monitoring device if the first key information and the second key information are the same;
a second health monitoring unit, configured to send the health monitoring data to the server; the server sends the health monitoring data to the second terminal device for display;
a second health monitoring unit, configured to send monitoring invitation information containing account information of the second terminal device 12 to the server; the server sends a download link of a data monitoring system to the second terminal device 12 if determining that the second terminal device has not downloaded and logged in to the data monitoring system based on the account information, so that the second terminal device may download and log in to the data monitoring system; the server sends the monitoring invitation information to the second terminal device if determining that the second terminal device has downloaded and logged in to the data monitoring system based on the account information; the server sends the health monitoring data to the second terminal device 12 for display after receiving an invitation acceptance instruction sent by the second terminal device.

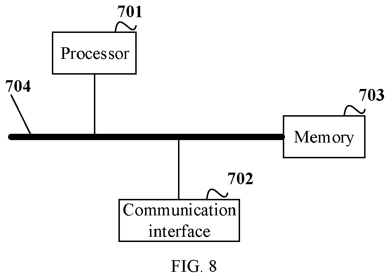

The present application also provides a terminal device, as shown in FIG. 8, it includes a processor 701, a communication interface 702, a memory 703, and a communication bus 704, and the processor 701, the communication interface 702 and the memory 703 communicate with each other through the communication bus 704;
the memory 703 stores a computer program, and the processor 701 implements the steps of any of the above methods when the program is executed by the processor 701.

The communication bus of the terminal device may be a peripheral component interconnect (PCI) bus or an extended industry standard architecture (EISA) bus. The communication bus may be divided into address bus, data bus, and control bus. For convenience of illustration, the bus is represented only by a thick line in the figure, but it does not mean that there is only one bus or one type of bus.

The communication interface 702 is used for the communication between the terminal device and other devices.

The memory may include random access memory (RAM) or non-volatile memory (NVM), for example, it may be at least one disk memory. Alternatively, the memory may also be at least one storage device located remotely from the processor.

The processor may be a general-purpose processor, including central processing unit and network processor (NP), or may also be a digital signal processing (DSP), special integrated circuit, field programmable gate array or programmable logic device, discrete gate or transistor logic device, and discrete hardware component.

The present application also provides a computer-readable storage medium, where a computer program executable by a terminal device is stored on the computer-readable storage medium, and the program is executed by the terminal device to implement the steps of any of the above methods when the program runs on the terminal device.

Although preferred embodiments of the present application have been described, those skilled in the art may make additional changes and modifications to these embodiments once they become aware of basic inventive concepts. Therefore, these appended claims are intended to be interpreted to include preferred embodiments and all changes and modifications falling within the scope of the present application.

It will be apparent to those skilled in the art that various modifications and variations can be made to the present application without departing from the spirit or scope of the present application. Thus, if these modifications and variations of the present application fall within the scope of the claims of the present application and their equivalents, the present application is also intended to include these modifications and variations.

## Claims

1. A health monitoring system, **characterized in that**, the system comprises a first terminal device of a first user, a second terminal device of a second user, a health monitoring device, and a server;
wherein the first terminal device is configured to obtain a unique activation code from the server and generate a QR code containing the unique activation code, and the server is configured to associate the unique activation code with first user information;
wherein the second terminal device is configured to obtain the unique activation code by scanning the QR code, awaken the health monitoring device, obtain identification information of the health monitoring device, and send the identification information of the health monitoring device and the unique activation code to the server;
wherein the server is configured to downlink the identification information to the first terminal device;
wherein the first terminal device is configured to establish a connection to the health monitoring device based on the identification information, receive health monitoring data sent by the health monitoring device, and display the health monitoring data.

2. The system according to claim 1, wherein the server verifies the identification information and the unique activation code before downlinking the identification information to the first terminal device, and associates the identification information of the health monitoring device with the first user information based on the unique activation code after the verification is passed, to realize the binding between the health monitoring device and the first user and downlink the identification information to the first terminal device.

3. The system according to claim 2, wherein the first terminal device is further configured to encrypt the unique activation code by using a first encryption/decryption algorithm to obtain first ciphertext information, and embed the first ciphertext information into the QR code;
wherein the second terminal device is configured to obtain the first ciphertext information by scanning the QR code, and decrypt the first ciphertext information by using the first encryption/decryption algorithm to obtain the unique activation code.

4. The system according to claim 1, wherein the health monitoring device is further configured to encrypt a first authentication parameter by using a second encryption/decryption algorithm to obtain second ciphertext information, and send first authentication request information containing the second ciphertext information to the first terminal device;
wherein the first terminal device is further configured to encrypt a second authentication parameter by using a third encryption/decryption algorithm if determining the successful authentication of the health monitoring device based on the first authentication request information to obtain third ciphertext information, and send second authentication request information containing the third ciphertext information to the health monitoring device;
wherein the health monitoring device is further configured to send the health monitoring data to the first terminal device if determining the successful authentication of the first terminal device based on the second authentication request information.

5. The system according to claim 4, wherein the first terminal device is further configured to obtain the second ciphertext information contained in the first authentication request information, and decrypt the second ciphertext information by using the second encryption/decryption algorithm to obtain the first authentication parameter; obtain first key information and corresponding first key identifier information in the first authentication parameter; obtain locally stored second key information corresponding to the first key identifier information; determine the successful authentication of the health monitoring device if the first key information and the second key information are the same;
wherein the health monitoring device is further configured to obtain the third ciphertext information contained in the second authentication request information, and decrypt the third ciphertext information by using the third encryption/decryption algorithm to obtain the second authentication parameter; obtain third key information and corresponding second key identifier information in the second authentication parameter; obtain locally stored fourth key information corresponding to the second key identifier information; determine the successful authentication of the first terminal device if the third key information and the fourth key information are the same.

6. The system according to claim 1, wherein the first terminal device is further configured to send the health monitoring data to the server;
wherein the server is further configured to send the health monitoring data to the second terminal device for display.

7. The system according to claim 6, wherein the first terminal device is further configured to send monitoring invitation information containing account information of the second terminal device to the server;
wherein the server is further configured to send a download link of a data monitoring system to the second terminal device if determining that the second terminal device has not downloaded and logged in to the data monitoring system based on the account information, the second terminal device may download and log in to the data monitoring system; send the monitoring invitation information to the second terminal device if determining that the second terminal device has downloaded and logged in to the data monitoring system based on the account information;
wherein the server is further configured to send the health monitoring data to the second terminal device for display after receiving an invitation acceptance instruction sent by the second terminal device.

8. A health monitoring method, **characterized in that**, the method is applied to a second terminal device and comprises:
obtaining the unique activation code by scanning the QR code, awakening the health monitoring device, obtaining identification information of the health monitoring device, and sending the identification information of the health monitoring device and the unique activation code to the server; the server downlinks the identification information to the first terminal device; the first terminal device establishes a connection to the health monitoring device based on the identification information, receives health monitoring data sent by the health monitoring device, and displays the health monitoring data; the QR code is a QR code generated by the first terminal device and containing the unique activation code.

9. The method according to claim 8, wherein the unique activation code is a universally unique identifier or a unique code generated by using a snowflake algorithm;
wherein the method further comprises: after the second terminal device sends the identification information of the health monitoring device and the unique activation code to the server; the server verifies the identification information and the unique activation code before downlinking the identification information to the first terminal device, wherein the verification is passed if the following conditions are met simultaneously: the health monitoring device corresponding to the current identification information is not activated and the health monitoring device does not expire; the current unique activation code is within the validity period and does not become invalid, and the current unique activation code within the validity period means that the time difference between the current time and the binding time of the unique activation code and the user information is less than the set time threshold; the unique activation code information is successfully decrypted.

10. A health monitoring method, wherein the method is applied to a first terminal device and comprises:
obtaining a unique activation code from a server and generating a QR code containing the unique activation code; establishing a connection to a health monitoring device based on identification information of the health monitoring device, receiving health monitoring data sent by the health monitoring device, and displaying the health monitoring data; a second terminal device obtains the unique activation code by scanning the QR code, awakens the health monitoring device, obtains the identification information of the health monitoring device, and sends the identification information to the server; the server downlinks the identification information to the first terminal device.

11. The method according to claim 10, wherein the method further comprises:
receiving first authentication request information sent by the health monitoring device, wherein the health monitoring device encrypts a first authentication parameter by using a second encryption/decryption algorithm to obtain second ciphertext information, and sends the first authentication request information containing the second ciphertext information to the first terminal device;
encrypting a second authentication parameter by using a third encryption/decryption algorithm if determining the successful authentication of the health monitoring device based on the first authentication request information to obtain third ciphertext information, and sending second authentication request information containing the third ciphertext information to the health monitoring device; the health monitoring device sends the health monitoring data to the first terminal device if determining the successful authentication of the first terminal device based on the second authentication request information;
wherein the determining the successful authentication of the health monitoring device based on the first authentication request information comprises:
obtaining the second ciphertext information contained in the first authentication request information, and decrypting the second ciphertext information by using the second encryption/decryption algorithm to obtain the first authentication parameter; obtaining first key information and corresponding first key identifier information in the first authentication parameter; obtaining locally stored second key information corresponding to the first key identifier information; determining the successful authentication of the health monitoring device if the first key information and the second key information are the same.

12. A health monitoring apparatus, wherein the apparatus is applied to a second terminal device and comprises:
a first health monitoring unit, configured to obtain the unique activation code by scanning the QR code, awaken the health monitoring device, obtain identification information of the health monitoring device, and send the identification information of the health monitoring device and the unique activation code to the server; the server downlinks the identification information to the first terminal device; the first terminal device establishes a connection to the health monitoring device based on the identification information, receives and displays health monitoring data sent by the health monitoring device; the QR code is a QR code generated by the first terminal device and containing the unique activation code.

13. A health monitoring apparatus, wherein the apparatus is applied to a first terminal device and comprises:
a second health monitoring unit, configured to obtain a unique activation code from a server and generate a QR code containing the unique activation code; establish a connection to a health monitoring device based on identification information of the health monitoring device, receive and display health monitoring data sent by the health monitoring device; a second terminal device obtains the unique activation code by scanning the QR code, awakens the health monitoring device, obtains the identification information of the health monitoring device, and sends the identification information to the server; the server downlinks the identification information to the first terminal device.

14. A terminal device, wherein it comprises a processor, a communication interface, a memory, and a communication bus, and the processor, the communication interface and the memory communicate with each other through the communication bus;
wherein the memory is configured to store a computer program;
wherein the processor is configured to implement the steps of the method according to any one of claims 8 or 9 or the steps of the method according to any one of claims 10 or 11 when executing the program stored in the memory.

15. A computer-readable storage medium, wherein a computer program is stored on the computer-readable storage medium, and the computer program is executed by a processor to implement the steps of the method according to any one of claims 8 or 9 or the steps of the method according to any one of claims 10 or 11.
